# NEW EUROPEAN PATENT SPECIFICATION

(11) **EP 0 367 787 B2**
(45) Date of publication and mention of the opposition decision: **27.01.1999**
(45) Mention of the grant of the patent: 22.11.1995
(21) Application number: 88906261.8
(22) Date of filing: 12.05.1988
(51) Int. Cl.: A61K 49/00, A61K 49/04

(54) **METHOD FOR ENHANCING THE SAFETY OF METAL-LIGAND CHELATES AS X-RAY CONTRAST AGENTS**
VERFAHREN ZUR ERHÖHUNG DER SICHERHEIT VON METALL-LIGANDCHELATEN ALS RÖNTGENSTRAHLEN-KONTRASTMITTEL
PROCEDE PERMETTANT D'AUGMENTER LA SECURITE DE CHELATES DE LIGAND METALLIQUE UTILISES COMME AGENTS D'IMAGERIE DE COMME AGENTS DE CONTRASTE DE RAYONS X

(30) Priority: 30.06.1987 US 68588
(43) Date of publication of application: 16.05.1990
(73) Proprietor: Mallinckrodt, Inc. (a Delaware corporation), St. Louis Missouri 63134 (US)
(72) Inventor: VANDERIPE, Donald, R., Lake St. Louis, MO 63367 (US)
(74) Representative: Eyles, Christopher Thomas
(86) International application number: US8801601
(87) International publication number: WO8900052

(56) References cited:
- EP-A- 130 934
- EP-A- 270 483
- EP-A- 0 258 616
- WO-A-90/03804
- GB-A- 736 432
- US-A- 4 647 447
- US-A- 4 686 104
- Holleman-Wiberg, Lehrbuch der Anorganischen Chemie, 1976, pp. 150-151

## Description

### BACKGROUND OF THE INVENTION

This invention relates to X-ray imaging complexes and more particularly to methods for reducing the toxicity thereof.

It has been found that physiologically well-tolerated complex salts formed from the anion of a complexing acid (ligand) and one or more central ions of an element with a atomic number of 21 to 29, 42, 44 or 57 to 70 (paramagnetic metal) and, optionally, also formed from one or more physiologically biocompatible cations of an inorganic and/or organic base or amino acid, are suitable for producing diagnostic media for use in X-ray diagnosis. We have referred to these materials as paramagnetic metal chelates. U.S. Patent 4,647,447 describes the use and the manufacture of paramagnetic metal chelates in detail.

However, it has been found that these paramagnetic chelates acutely reflect more toxicity when injected in high concentration or at rapid rates. Generally, this toxicity has been manifested as strong convulsions.

Believing this toxicity to stem from the absorption of free paramagnetic metals in the blood, it has been the previous practice to reduce such toxicity by formulating with an additive of excess ligand such as EDTA as its sodium and/or calcium salts. These additives were employed as scavengers for the paramagnetic metal in the manner disclosed by Bernard Osler et al. in Toxicology and Applied Pharmacology, Volume 5, Pages 142-162 published in 1963 under title of "Safety Evaluation Studies of Calcium EDTA".

However, a method for reducing such toxicity without the need for employing excess ligand and without having to correlate the amount of excess ligand to the projected amount of free metal in the blood would be a substantial advancement in the art.

### SUMMARY OF THE INVENTION

It is an object of this invention to provide enhanced safety in X-ray contrast imaging by reducing the toxicity of heavy metal chelates.

Upon further study of the specification and appended claims, further objects and advantages of this invention will become apparent to those skilled in the art.

It has been found that adding calcium ions in substantially less than stoichiometric proportions to the metal-ligand chelates used in X-ray contrast formulations will substantially reduce the acute intravenous toxicity thereof.

According to the present invention there is provided the use for the manufacture of a composition for X-ray diagnosis of a metal-ligand complex formed from the anion of a complexing acid and having a central ion of an element with an atomic number of 21 to 29, 42, 44 or 57 to 70 in admixture with calcium ions in an amount effective to reduce the toxicity of the metal-ligand complex and of from about 1 to about 25% of the stoichiometric amount required by the metal-ligand complex, the calcium ions being provided in the form of an inorganic calcium salt or a mixture of an inorganic calcium salt with one or both of an organic calcium salt and a calcium-sodium salt of a chelant ligand.

### DESCRIPTION OF THE PREFERRED EMBODIMENTS

In accordance with the present invention, calcium in the form of calcium gluconate, calcium chloride or other suitable organic or inorganic salts and including suitable soluble calcium forms of the chelant/ligand used to complex paramagnetic and/or heavy metals are added to the product formulation to be used for X-ray contrast imaging.

The calcium ions are added at levels ranging from 1-25% of stoichiometic based on the chelant/ligand concentration. In this specification, as can be seen from the Table in Example 2 below, a 1:1 Ca:metal complex molar ratio corresponds to a 100% stoichiometric amount of calcium required by the metal-ligand complex.

The amount of calcium added is determined individually for each formulation and will depend on the calcium chelation potential of the formulation. The calcium can be added in a single form, e.g. calcium chloride, or as mixtures, e.g. calcium chloride and calcium gluconate.

The paramagnetic and/or heavy metal chelates to which calcium is to be added are complex salts formed from the anion of a complexing acid and a central ion of an element with an atomic number of 21 to 29, 42, 44 or 57 to 70 and, optionally, also formed from one or more physiologically bio-compatible salts of inorganic and/or organic bases or amino acids. They are suitable for producing diagnostic media which are useful in X-ray diagnosis. The central ion preferably is the divalent or trivalent ion of elements with an atomic number of 21 to 29, 42, 44 and 57 to 70. Suitable ions for example, chromium 3, manganese 2, iron 3, iron 2, cobalt 2, copper 2, praseodymium 3, neodymium 3, samarium 3, ytterbium 3 and because of their very strong magnetic moments gadolinium 3, terbium 3, dysprosium 3, holmium 3, and erbium 3 are preferred.

The central ion should preferably be derived from an element with a higher atomic number to achieve a sufficient absorption of X-rays. It has been found that diagnostic media containing a physiologically well-tolerated complex salt with central ions of elements with atomic number of 57 to 70 are particularly suitable for this purpose. These include, for example, lanthanum 3, and the above-mentioned ions of the lanthanide group.

The action of citric acid, ethylenediamine-tetracetic acid (EDTA) and similar ligands to complex with ionic calcium when injected in-vivo into the bloodstream and inducing tetanic convulsions has long been appreciated. Calcium, administrated as a chloride salt or as calcium gluconate is known to be effective in counteracting these convulsions. However, such teachings have previously found little application in reducing the toxicity of X-ray contrast agents. Among the prior art reasons for not resorting to ionic calcium in such applications may have been that ionic calcium if added at stoichiometric amounts would have been detrimental because these hypertonic solutions would then have provided excessive amounts of calcium upon injection into the bloodstream. Additionally, calcium complexation by iodinated X-ray contrast media was not significant compared to the newer paramagnetic complexes.

Complex paramagnetic chelates and heavy metals complexed to chelant ligands as previously stated have limited clinical utility at increasing dosages because of the toxicity created therefrom. Toxicity which is usually greater when the agents are injected rapidly and/or at more concentrated levels is generally attributed to the in-vivo release of the heavy metal. Therefore, the addition of excess ligand to the formulation to bind any "free" metal in the injectable is felt to be of value. However, the effective scavaging amount of excess ligand at, for example, 15% excess sodium salt fails to enhance the safety of the Na₂ GdDTPA. Addition of 15% excess ligand as the CaNa₃ DTPA to Na₂ GdDTPA increased the intravenous LD₅₀ about 20%. Although this level of excess ligand would provide about 4mg/ml of calcium, the following examples show that further enhancement of safety can be expected by adjusting the level of calcium simply by addition of calcium chloride, i.e. without need for excess ligand.

The toxicity of the preferred embodiments of the present invention are measured by lethal dose (LD) values which are approximations of the doses at which the specimen animals die. Exemplary lethal dose values for the present invention are seen in the examples set forth below:

### EXAMPLE 1

The intravenous LD₅₀ for calcium chloride (CaCl₂) in the mouse is reported to be 42 mg/kg (RTECS). This calculates to about 15 mg/kg of calcium or about 0.3 mg of calcium for a 20 gram mouse. When calcium chloride was added to 0.68M disodium gadolinium diethylenetriamine-pentaacetic acid (Na₂GdDTPA) at 130 mg/kg (6.5 mg/ml) or 260 mg/kg (13.0 mg/ml) the lethal effects of the Na₂GdDTPA were greatly diminished even though these levels of added calcium would provide 47 and 94 mg/kg, i.e. 0.94 and 1.88 mg respectively to a 20 gram mouse. These values are 3.1 and 6.2 times higher than the i.v. LD₅₀ of calcium administered as CaCl₂. Whereas 4 of 4 mice given 13.6 mMol/kg of Na₂GdDTPA alone died, only 1 of 4 mice died at those doses of Na₂GdDTPA with 2.34 mg/ml of added calcium and 2 of 4 died at the 4.68 mg/ml level of added calcium. Clearly then the 0.68 m Na₂GdDTPA solution must complex a substantial amount of the added calcium in a way to block the calcium's in-vivo toxicity.

Conversely, the calcium added to the Na₂GdDTPA formulation blocks the in vivo calcium complexation by Na₂GdDTPA and thereby reduces its toxicity, i.e. prevents tetanic convulsions and death. Clearly this protective effect of added calcium must be balanced to the calcium complexing potential of the paramagnetic contrast agent.

### EXAMPLE 2

Addition of calcium chloride at 430 mg/kg (21.5 mg/ml) and 860 mg/kg (43 mg/ml), i.e. at 155 and 310 mg of calcium /kg, results in doses of 3.1 and 6.2 mg of calcium per 20 gram mouse. These doses of calcium added to 0.68 m Na₂GdDTPA were not protective and did not enhance the safety of the Na₂GdDTPA formulation. All mice injected with 0.65m Na₂GdDTPA at those two dose levels of calcium died. It may be inferred that these levels of added calcium were excessive and exceeded the calcium binding optimum of the solution and that death from calcium toxicity ensued.

In these Examples 1 and 2 (see Table 1) it is shown that calcium added to 0.68 M Na₂GdDTPA as calcium chloride at concentrations of 6.5, 13.0, 21.5 and 43 mg/ml of solution and which would result in concentrations of calcium of 2.34 mg, 4.68 mg, 7.74 mg and 15.48 mg per millilitre respectively, provided different levels of protection against the toxicity of Na₂GdDTPA. On a stoichiometric basis the four added calcium levels approximate 9%, 17%, 29% and 57% respectively of the 0.68 m concentration of Na₂GdDTPA. Based on this data, concentrations of added calcium of 30-60% stoichiometric to that of the subject formulation are excessive and do not enhance the safety. However, concentrations of added calcium of 9-17% stoichiometry to the subject formulation were protective based on acute toxicity determinations.

Clearly the optimum amount of calcium to be added will vary based on the ligand chosen and its concentration in the formulation.

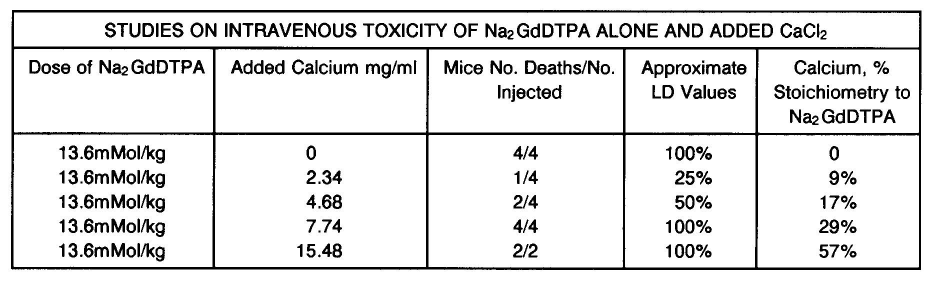

## Claims

1. The use for manufacture of a composition for in vivo X-ray diagnosis of a metal-ligand complex formed from the anion of a complexing acid and having a central ion of an element with an atomic number of 21 to 29, 42, 44 or 57 to 70, in admixture with calcium ions in an amount effective to reduce the toxicity of the metal-ligand complex and of from about 1 to about 25% of the stoichiometric amount required by the metal-ligand complex, the calcium ions being provided in the form of an inorganic calcium salt or a mixture of an inorganic calcium salt with one or both of an organic calcium salt and a calcium-sodium salt of a chelant ligand.

2. The use according to claim 1 of a metal-ligand complex that contains a central ion of an element with an atomic number of 57 to 70.

3. The use according to claim 1 or claim 2, wherein the calcium ions are provided in the form of calcium chloride.

4. The use according to any one of claims 1 to 3, wherein the metal-ligand chelate complex is in the form of a salt.

5. The use according to any one of claims 1 to 3, wherein the metal-ligand chelate complex is in non-ionic form.

6. The use according to any one of claims 1 to 5, wherein the calcium ions are present in an amount of from about 3 to about 15% of the stoichiometric amount required by the metal-ligand complex.

## Patentansprüche

1. Verwendung eines Metall-Ligand-Komplexes aus einem Anion einer komplexierenden Säure, das ein Zentralion eines Elements mit einer Atomzahl von 21 bis 29, 42, 44 oder 57 bis 70 hat, im Gemisch mit Calciumionen in einer zur Reduzierung der Toxizität des Metall-Ligand-Komplexes wirksamen Menge von etwa 1 bis etwa 25 % der für den Metall-Ligand-Komplex erforderlichen stöchiometrischen Menge, wobei die Calciumionen in der Form eines anorganischen Calciumsalzes oder eines Gemisches eines anorganischen Calciumsalzes mit einem organischen Calciumsalz oder einem Calcium-Natriumsalz eines chelatbildenden Liganden oder mit beiden vorgesehen sind, für die Herstellung einer Zusammensetzung für die Röntgenstrahldiagnose in vivo.

2. Verwendung nach Anspruch 1 eines Metall-Ligand-Komplexes. der ein Zentralion eines Elements mit einer Atomzahl von 57 bis 70 enthält.

3. Verwendung nach Anspruch 1 oder Anspruch 2, bei der die Calciumionen in Form von Calciumchlorid vorgesehen werden.

4. Verwendung nach einem der Ansprüche 1 bis 3, bei der der Metall-Ligand-Komplex die Form eines Salzes hat.

5. Verwendung nach einem der Ansprüche 1 bis 3, bei der der Metall-Ligand-Chelatkomplex eine nicht-ionische Form hat.

6. Verwendung nach einem der Ansprüche 1 bis 5, bei der die Calciumionen in einer Menge von etwa 3 bis 15 % der für den Metall-Ligand-Komplex erforderlichen stöchiometrischen Menge vorliegen.

## Revendications

1. Utilisation, pour la fabrication d'une composition pour le diagnostic radiographique in vivo, d'un complexe métal-ligand formé de l'anion d'un acide complexant et ayant un ion central d'un élément d'un nombre atomique de 21 à 29, 42, 44 ou 57 à 70, en mélange avec des ions calcium en une quantité efficace pour réduire la toxicité du complexe métal-ligand et d'environ 1 à environ 25 % de la quantité stoechiométrique requise par le complexe métal-ligand, les ions calcium étant fournis sous forme d'un sel de calcium inorganique ou d'un mélange d'un sel de calcium inorganique avec un sel de calcium organique ou un sel de calcium-sodium d'un ligand chélatant, ou les deux,

2. Utilisation selon la revendication 1 d'un complexe métal-ligand qui contient un ion central d'un élément d'un nombre atomique de 57 à 70.

3. Utilisation selon la revendication 1 ou la revendication 2, dans laquelle les ions calcium sont fournis sous forme de chlorure de calcium.

4. Utilisation selon l'une quelconque des revendications 1 à 3, dans laquelle le complexe chélate métal-ligand est sous forme d'un sel.

5. Utilisation selon l'une quelconque des revendications 1 à 3, dans laquelle le complexe chélate métal-ligand est sous forme non ionique.

6. Utilisation selon l'une quelconque des revendications 1 à 5, dans laquelle les ions calcium sont présents en une quantité d'environ 3 à environ 15 % de la quantité stoechiométrique requise par le complexe métal-ligand.
